# EUROPEAN PATENT SPECIFICATION

(11) **EP 2 149 382 B1**
(45) Date of publication and mention of the grant of the patent: **01.08.2012**
(21) Application number: 08739206.4
(22) Date of filing: 28.03.2008
(51) Int. Cl.: A61K 45/00, A61L 27/00, A61K 6/00, A61K 8/49, A61K 31/366, A61K 35/32, A61P 1/02, A61Q 11/00

(54) **DENTINOGENESIS INDUCER AND DENTINOGENESIS PULP-CAPPING MATERIAL**
MITTEL ZUR FÖRDERUNG DER DENTINOGENESE UND MATERIAL ZUR BEDECKUNG VON DENTINOGENER PULPA
PROMOTEUR DE LA DENTINOGENÈSE ET MATIÈRE DE COIFFAGE PULPAIRE DENTINOGÉNIQUE

(30) Priority: 30.03.2007 JP 2007094213
(43) Date of publication of application: 03.02.2010
(73) Proprietor: Organ Technologies Inc., Tokyo 101-0048 (JP)
(72) Inventor: KUBOKI, Takuo, Okayama-shi Okayama 700-8558 (JP); ONO, Mitsuaki, Okayama-shi Okayama 700-8558 (JP); SONOYAMA, Wataru, Okayama-shi Okayama 700-8558 (JP); FUJISAWA, Takuo, Okayama-shi Okayama 700-8558 (JP); SHIMONO, Kengo, Okayama-shi Okayama 700-8558 (JP); OSHIMA, Masamitsu, Okayama-shi Okayama 700-8558 (JP); OKAMOTO, Yosuke, Okayama-shi Okayama 700-8558 (JP)
(74) Representative: Wasner, Marita
(86) International application number: PCT/JP2008/056088
(87) International publication number: WO 2008/120720

(56) References cited:
- WO-A1-2006/009291
- WO-A1-2006/009291
- MUNDY G ET AL: "STIMULATION OF BONE FORMATION IN VITRO AND IN RODENTS BY STATINS", SCIENCE, AMERICAN ASSOCIATION FOR THE ADVANCEMENT OF SCIENCE, WASHINGTON, DC; US, vol. 286, no. 5446, 3 December 1999 (1999-12-03), pages 1946-1949, XP000999135, ISSN: 0036-8075, DOI: DOI:10.1126/SCIENCE.286.5446.1946
- BAEK KI HYUN ET AL: "The effect of simvastatin on the proliferation and differentiation of human bone marrow stromal cells", JOURNAL OF KOREAN MEDICAL SCIENCE, vol. 20, no. 3, June 2005 (2005-06), pages 438-444, XP002612186, ISSN: 1011-8934
- MIN K S ET AL: "Simvastatin Promotes Odontoblastic Differentiation and Expression of Angiogenic Factors via Heme Oxygenase-1 in Primary Cultured Human Dental Pulp Cells", JOURNAL OF ENDODONTICS, LIPPINCOTT WILLIAMS & WILKINS, PHILADELPHIA, PA, US, vol. 36, no. 3, 1 March 2010 (2010-03-01), pages 447-452, XP026912185, ISSN: 0099-2399, DOI: DOI:10.1016/J.JOEN.2009.11.021 [retrieved on 2010-01-25]
- OKAMOTO Y ET AL: "Simvastatin Induces the Odontogenic Differentiation of Human Dental Pulp Stem Cells In Vitro and In Vivo", JOURNAL OF ENDODONTICS, LIPPINCOTT WILLIAMS & WILKINS, PHILADELPHIA, PA, US, vol. 35, no. 3, 1 March 2009 (2009-03-01), pages 367-372, XP026032497, ISSN: 0099-2399, DOI: DOI:10.1016/J.JOEN.2008.11.024 [retrieved on 2009-02-27]
- BENOIT D.S.W. ET AL: 'Synthesis and characterization of a fluvastatin-releasing hydrogel delivery system to modulate hMSC differentiation and function for bone regeneration' BIOMATERIALS vol. 27, no. 36, 2006, pages 6102 - 6110, XP008119974
- ONAKA K. ET AL.: 'Statin to BMP' CLINICAL CALCIUM vol. 14, no. 2, 2004, pages 286 - 288

## Description

### [Technical Field]

The present invention relates to a dentinogenesis inducer and dentinogenesis pulp-capping material, which are effectively used for the formation (regeneration) of dentin. The present invention also relates to a method of producing odontoblasts useful for dental regenerative therapy and to the application thereof.

### [Background Art]

When a tooth decays, the tooth is treated by the following procedures depending on the degree of the decay: a procedure to protect pulp instead of removing the pulp (direct pulp capping); a procedure to remove only a portion of the pulp (coronal pulp) and preserve radicular pulp (vital pulpotomy); and a procedure to completely remove the pulp and seal the hollow chamber with metal or resin (pulpectomy). However, there is a problem: once the pulp is removed, the supply of nutrients to the tooth is discontinued, which weakens dentin, and there is no nerve to indicate any pain, and thus subjective symptoms cannot be perceived when the decay advances again, which worsens the condition. Accordingly, in recent years, preservation of as much of the pulp as possible is considered preferable in order to maintain the health of the teeth.

In order to preserve the pulp and maintain its function, indirect pulp-capping material or direct pulp-capping material is used according to the extent of the damage of dentin and the pathological condition of the pulp. Indirect pulp-capping material is used when the pulp is not exposed although the dentin is broken, and direct pulp-capping material is used when the pulp is exposed and/or when a portion of the dentin is severed for the treatment. As direct pulp-capping material, calcium hydroxide preparations and formocresol preparations are conventionally used. However, calcium hydroxide does not have an effect of inducing odontoblasts, and thus the induction of dentinogenesis cannot be expected therefrom. As a substance having a dentinogenesis inducing effect, the following substances have been so far reported: bovine blood extract (Patent Documents 1), polysaccharides such as N-acetylglucosamine (Patent Documents 2), osteogenic factors such as BMP-2 (bone morphogenetic protein) (Patent Documents 3), growth factors such as TGF-β (non-patent document 1), etc. However, the use of bovine blood extract involves risks including prion infections such as mad cow disease, unknown virus infections, etc., and thus it is difficult to actually use bovine blood extract in humans. The use of N-acetylglucosamine may also adversely affect humans due to an unknown infection source and contaminants because the N-acetylglucosamine is derived from living tissues such as the husks of insects and crustacean shells as raw materials. With a method that uses osteogenic factors such as BMP-2 (bone morphogenetic protein) and TGF-β, the preparation process of recombinant protein of human origin is complex and has a low yield, and thus the resulting product may be extremely expensive.

Meanwhile, statin, i.e., an HMG-CoA reductase inhibitor, is a drug that has generally been used in many patients as a drug for hyperlipidemia, and the statin has been reported to have an effect of inducing osteogenic differentiation of mesenchymal stem cells and osteoblasts and an effect of accelerating the healing of bone fractures (Non-patent Document 2). However, it has been unknown whether an HMG-CoA reductase inhibitor has an effect of inducing differentiation of odontoblast precursor cells such as dental pulp stem cells into odontoblasts, i.e., it has been unknown whether an HMG-CoA reductase inhibitor has an effect of inducing the formation (regeneration) of dentin.
[Patent Document 1] Japanese Unexamined Patent Application Publication No. 2002-363084 A
[Patent Document 2] Japanese Unexamined Patent Application Publication No. 06-256132 A
[Patent Document 3] Japanese Unexamined Patent Application Publication No. 06-340555 A
[Non-patent Document 1] Sloan AJ, Smith AJ.: Stimulation of the dentine-pulp complex of rat incisor teeth by transforming growth factor-beta isoforms 1-3 in vitro. Arch Oral Biol., 44 (2) :149-56, 1999.
[Non-patent Document 2] Mundy G, Garrett R, Harris S, et al. Stimulation of bone formation in vitro and in rodents by statins. Science, 286:1946-1949, 1999.

### [Disclosure of Invention]

### Technical Problem

An object of the present invention is to provide technology for efficiently inducing the differentiation of cells capable of differentiating into odontoblasts such as dental pulp stem cells (hereinafter such cells are referred to as "odontoblast precursors") into odontoblasts so as to form (regenerate) dentin. More specifically, the present invention aims to provide a dentinogenesis inducer having an effect of inducing differentiation of "odontoblast precursors" into odontoblasts and capable of being suitably used as a pretreatment agent for a carious portion and/or a surface of exposed pulp (exposed pulp surface). Another object of the present invention is to provide dentinogenesis pulp-capping material capable of stimulating differentiation of "odontoblast precursors" into odontoblasts and inducing dentinogenesis by covering and protecting the exposed pulp surface when the pulp is treated for caries therapy. Further, the present invention aims to provide dental treatment material necessary for regeneration of dentin, using "odontoblast precursors".

### Solution to Problem

The present inventors conducted intensive studies in an attempt to solve the above-described problem, and found that culturing dental pulp stem cells (DPSCs) connected with dentinogenesis in the presence of statin, i.e., an HMG-CoA reductase inhibitor, aids the inhibition of the proliferation of DPSCs and significantly increases gene expression of dentin sialophosphoprotein (DSPP), i.e., an odontoblast-specific marker, and osteocalcin (OCN), i.e., a specific marker of calcification. Additionally, the present inventors found that the DPSC proliferation inhibiting effect of statin disappears as a result of the supply of mevalonate whose production is inhibited by an HMG-CoA reductase inhibitor in the cholesterol synthesis pathway. Specifically, these findings indicate that a HMG-CoA reductase inhibitor having an effect of inhibiting HMG-CoA reductase in the cholesterol synthesis pathway has an effect of inducing differentiation of dental pulp stem cells into odontoblasts and thereby promoting dentinogenesis, in other words, that an HMG-CoA reductase inhibitor is useful as an active ingredient of a dentinogenesis inducer and/or dentinogenesis pulp-capping material. Further, the above findings indicate that the use of an HMG-CoA reductase inhibitor enables the provision of a method capable of transforming conventional dental treatments in which infected tissues are removed and replaced with artificial material into medical treatments in which infected/removed dentin is regenerated (regeneration of reparative dentin, regeneration of a dental root).

The present invention is completed based on the above knowledge, and has the following structure.

### I. Dentinogenesis Inducer

(I-1) A dentinogenesis inducer containing an HMG-CoA reductase inhibitor as an active ingredient.
(1-2) The dentinogenesis inducer according to (I-1), wherein the HMG-CoA reductase inhibitor is at least one selected from the group consisting of pravastatin, simvastatin, fluvastatin, atorvastatin, rosuvastatin, mevastatin, lovastatin, pitavastatin, and salts thereof.
(I-3) Use of the HMG-CoA reductase inhibitor as a dentinogenesis inducer.
(1-4) Use of the HMG-CoA reductase inhibitor for manufacture of a dentinogenesis inducer.
(1-5) The use according to (1-3) or (1-4), wherein the HMG-CoA reductase inhibitor is at least one selected from the group consisting of pravastatin, simvastatin, fluvastatin, atorvastatin, rosuvastatin, mevastatin, lovastatin, pitavastatin, and salts thereof.

### II. Dentinogenesis Pulp-capping Material

(II-1) Dentinogenesis pulp-capping material containing an HMG-CoA reductase inhibitor as an active ingredient.
(II-2) The dentinogenesis pulp-capping material according to (II-1), wherein the HMG-CoA reductase inhibitor is at least one selected from the group consisting of pravastatin, simvastatin, fluvastatin, atorvastatin, rosuvastatin, mevastatin, lovastatin, pitavastatin, and salts thereof.
(II-3) Use of the HMG-CoA reductase inhibitor as an active ingredient of dentinogenesis pulp-capping material.
(11-4) Use of the HMG-CoA reductase inhibitor for manufacture of dentinogenesis pulp-capping material.
(II-5) The use according to (II-3) or (II-4), wherein the HMG-CoA reductase inhibitor is at least one selected from the group consisting of pravastatin, simvastatin, fluvastatin, atorvastatin, rosuvastatin, mevastatin, lovastatin, pitavastatin, and salts thereof.

### III. Method of Producing Odontoblast, and Application of Odontoblast

(III-1) A method of producing an odontoblast comprising a step of culturing a cell capable of differentiating into an odontoblast in the presence of an HMG-CoA reductase inhibitor.
(III-2) The method of producing an odontoblast according to (III-1), wherein the cell capable of differentiating into an odontoblast is at least one member selected from the group consisting of dental pulp stem cells, stem cells from apical papilla, and odontoblast precursor cells.
(III-3) The method of producing an odontoblast according to (III-1) or (III-2), comprising culturing in the presence of an HMG-CoA reductase inhibitor at a concentration of 0.1-10 µM.
(III-4) The method of producing an odontoblast according to any one of (III-1) to (III-3), wherein the HMG-CoA reductase inhibitor is at least one selected from the group consisting of pravastatin, simvastatin, fluvastatin, atorvastatin, rosuvastatin, mevastatin, lovastatin, pitavastatin, and salts thereof.
(III-5) Dental treatment material containing the odontoblast obtained by using the method according to any one of (III-1) to (III-4) .

### [Advantageous Effect of Invention]

The dentinogenesis inducer of the present invention is suitable as a pretreatment agent particularly for filling restoration of a carious portion and/or a surface of exposed pulp (exposed pulp surface), and the use thereof for pretreatment of such restoration helps induce the formation (regeneration) of dentin in the interface between a restoration and a carious portion and/or exposed pulp surface. Further, the dentinogenesis pulp-capping material is capable of protecting pulp when used as coating material (tooth coating material) that directly covers the exposed pulp surface in caries treatment, and is also capable of inducing dentinogenesis (regeneration) by stimulating differentiation of cells capable of differentiating into odontoblasts such as dental pulp stem cells into odontoblasts. Additionally, the odontoblast produced by the method of the present invention is useful as tooth regeneration material for odontotherapy.

### [Detailed Description of Preferred Embodiment]

### (I) Dentinogenesis inducer

A dentinogenesis inducer of the present invention is characterized by containing an HMG-CoA reductase inhibitor as an active ingredient.

An HMG-CoA reductase inhibitor is a substance that has an effect of inhibiting the effects of an HMG-CoA reductase and thereby preventing the production of mevalonic acid in the cholesterol synthesis pathway in the body. Note that the HMG-CoA reductase (3-hydroxy 3-methylglutaryl-CoA reductase) is an enzyme required to produce mevalonic acid by reducing HMG-CoA in the process of cholesterol biosynthesis. Accordingly, because the HMG-CoA reductase inhibitor having the above-mentioned effect inhibits production of mevalonic acid, suppresses cholesterol biosynthesis, and lowers cholesterol, the inhibitor has been widely used as a drug for hyperlipidemia.

Any HMG-CoA reductase inhibitors may be used insofar as the inhibitors inhibit the effect of HMG-CoA reductase and have an effect of inhibiting generation of mevalonic acid. Although there is no limit, specific examples of HMG-CoA reductase inhibitors may include a water-soluble statin, namely, pravastatin; fat-soluble statins, namely, simvastatin, fluvastatin, atorvastatin, rosuvastatin, pitavastatin, mevastatin, lovastatin; and their salts. Note that pravastatin is available as Mevalotin (brand name) from Daiichi Sankyo Company, Limited; simvastatin as Lipovas (brand name) from Banyu Pharmaceutical Co., Ltd.; fluvastatin as Lescol (brand name) from Novartis Pharma K.K.; atrovastatin as Lipitor (brand name) from Astellas Pharma, Inc.; rosuvastatin as Crestor (brand name) from Shionogi & Co., Ltd.; pitavastatin as Livalo (brand name) from Kowa Company Ltd.; mevastatin from Calbiochem Ltd.; and lovastatin as Mevacor (brand name) from Merk, Ltd.

Among these HMG-CoA reductase inhibitors, mevastatin and lovastatin are lactone type inhibitors. These inhibitors themselves do no have HMG-CoA reductase inhibitory activities; however, they are converted into an acid type inside of the body and consequently will have HMG-CoA reductase inhibitory activities. Accordingly, the HMG-CoA reductase inhibitors used in the present invention also include substances that demonstrate HMG-CoA reductase inhibitory activities only inside of the body.

Any type may be suitably used as the dentinogenesis inducer of the present invention insofar as the inducer contains an effective amount of an HMG-CoA reductase inhibitor described above. The HMG-CoA reductase inhibitor described above may be prepared in forms of, for example, aqueous liquid formulations in which the HMG-CoA reductase inhibitor is dissolved or dispersed in water, or emulsions in which the HMG-CoA reductase inhibitor is emulsified using a surfactant.

When the dentinogenesis inducer of the present invention is formed in aqueous liquid formulations or emulsions, it is desirable to prepare the HMG-CoA reductase inhibitor such that the concentration of HMG-CoA reductase inhibitor at the time of use is normally 0.1 to 10 µM, preferably 0.2 to 5 µM, more preferably 0.5 to 2 µM. In other words, it suffices insofar as the concentration of HMG-CoA reductase inhibitor used during dentinogenesis is within the above ranges, and the concentration of HMG-CoA reductase inhibitor contained in the dentinogenesis inducer is not limited thereto. Normally, in view of the fact that the concentration is diluted during use, it is preferable that the HMG-CoA reductase inhibitor is contained at a concentration higher than those described above.

Other ingredients may be blended as desired into the dentinogenesis inducer within limits that do not impair the effects of the present invention. Examples of such other ingredients may include metallic fluorides having an anticaries effect such as sodium fluoride and silver diammine fluoride (brand name: Saforide); formocresol; and antibiotic substances and antimicrobial substances effective against obligate anaerobic bacteria. Additionally, the dentinogenesis inducer may contain bone osteogenic factor (bone morphogenic protein (BMP)-2, 4, and 7) having a dentinogenesis inducing effect, human basic fibroblast growth factor (bFGF), transforming growth factor (TGFβ), connective tissue growth factor (CTGF), hepatocyte growth factor (HGF), insulin-like growth factor (IGF-I, II), Sonic hedgehog (SHH), etc. as a biofunctional molecule having a potential for regenerating dentin. Further, the dentinogenesis inducer may also contain bone marrow-derived mesenchymal stem cells (BMSCs), dental pulp stem cells (DPSCs), stem cells from apical papilla (SCAP), etc. as cell components (Sonoyama W., Liu Y., Fang D., Yamaza T., Seo B-M et al (2006) Mesenchymal Stem Cell-Mediated Functional Tooth Regeneration in Swine. PLOS ONE 1(1): e79. doi: 10.1371/journal.pone.0000079.). Still further, carrier material such as hydroxyapatite, α- or β-TCP, adhesive resin, dental cement (glass ionomer, carboxylate cement, etc.), atelocollagen, and hyaluronic acid; and when pulp inflammation is severe, medical agents having anti-inflammatory effect such as phenol-camphor (CC), anti-inflammatory agents, etc., may also be used.

Additionally, the present invention may also be used in combination with drugs such as calcium hydroxide preparations, which have conventionally been used as direct pulp capping agents (agents that protect pulp tissue when a portion of the pulp is exposed); and with drugs such as zinc oxide eugenol formulations, zinc oxide creosote formulations, etc., which have been used as indirect pulp capping agents (agents used for blocking extraneous stimulation and sterilization when dentin is thin).

Within limits that do not impair the effects of the present invention, the dentinogenesis inducer of the present invention may further be blended with additives, as needed, such as organic solvents (for example, ethanol, isopropyl alcohol, propylene glycol, acetone, methyl ethyl ketone, etc.); stabilizers (for example, polyhydric alcohols such as glycerin, and ethylene glycol, macrogol, etc.); and preservatives (for example, p-hydroxybenzoic esters, benzalkonium chloride, etc.), or blended with other medically effective ingredients such as disinfectants, antibiotics, or anti-inflammatory agents.

The dentinogenesis inducer of the present invention in the form of a liquid formulation is directly used for application to a tooth, in particular, an exposed pulp surface (pulp chamber opening, a cut surface of the pulp, etc.) during caries treatment. Specifically, the inducer may be sprayed or applied to a target portion (exposed pulp surface) of a tooth surface in the oral cavity, or may be soaked in absorbent cotton or the like so as to be applied to a target portion as is or to fill a target portion therewith. In the above manner, the dentinogenesis inducer of the present invention efficiently penetrates through and infiltrates the exposed pulp surface into pulp cells, in particular, pulp stem cells and other cells (odontoblast precursors) capable of differentiating into odontoblasts, and allows stimulation of the differentiation of these cells into odontoblasts, i.e., induction of dentinogenesis.

Additionally, the shape (form) of the dentinogenesis inducer of the present invention is not particularly limited. For example, it may be in the form of aqueous liquid
formulations or emulsions as described above, or may be in the form of gels, creams, or ointments. When formulated in the form of gels, creams, or ointments, the application to the target portion (exposed pulp surface) of a tooth surface will be easy and the target portion can be filled with the dentinogenesis inducer. As a result, the retention on the exposed pulp surface is improved, and the effect of HMG-CoA reductase inhibitor can be more effectively provided. Note that when formulated in the form of aqueous liquid formulations or emulsions as described above, the dentinogenesis inducer may be blended with a thickener so as to impart viscosity thereto.

Note that types of thickeners are not particularly limited; however, examples of thickeners may include colloidal silica, fumed silica, aluminum oxide particles, montmorillonite particles, hydroxyapatite, α- or β-TCP, adhesive resin, dental cement (glass ionomer, carboxylic acid cement, etc.), atelocollagen, hyaluronic acid, gelatin hydrogel, etc. These may be used singly or in combination of two or more.

Additionally, the dentinogenesis inducer may be in the form of powders or granules. In such a case, the dentinogenesis inducer may be used by being dissolved, dispersed, or mixed in a suitable solvent such as water at the time of use.

The dentinogenesis inducer of the present invention is suitably used as a pretreatment agent during treatments such as filling restoration of a carious portion and/or an exposed pulp surface. The use of the dentinogenesis inducer of the present invention for pretreatment during the above-described treatment causes the HMG-CoA reductase inhibitor, which is an active ingredient, to penetrate through and infiltrate the exposed pulp surface into the "odontoblast precursors", thereby stimulating differentiation of these cells into odontoblasts and inducing dentinogenesis. As a result, the durability at the adhesive interface between a tooth and a restoration is improved.

### (II) Dentinogenesis Pulp-Capping Material

For the purpose of effectively providing the effect of the HMG-CoA reductase inhibitor serving as an active ingredient, the above-mentioned dentinogenesis inducer may be prepared as a dental material in a form supported by a carrier so as to retain and maintain the active ingredient on an affected area (exposed pulp surface) for a period sufficient to induce differentiation of "odontoblast precursors" into odontoblasts and form dentin. The "dentinogenesis pulp-capping material" of the present invention refers to such a dental material, and is characterized by containing the HMG-CoA reductase inhibitor as an active ingredient.

A specific example of the dentinogenesis pulp-capping material includes one in a form in which the HMG-CoA reductase inhibitor, preferably, the above-mentioned dentinogenesis inducer in the form of liquids, gels, creams or ointments comprising the HMG-CoA reductase inhibitor as an active ingredient is immobilized or impregnated in a structure such as a monofilament, film, fiber aggregate, sponge, particle, etc.

It is desirable to prepare the amount of HMG-CoA reductase inhibitor in the dentinogenesis pulp-capping material such that the concentration of HMG-CoA reductase inhibitor at the time of use is usually 0.1 to 10 µM, preferably 0.2 to 5 µM, more preferably 0.5 to 2 µM. In other words, it is prepared in a ratio in which the concentration of HMG-CoA reductase inhibitor used at the time of dentinogenesis will be within the above ranges.

With the dentinogenesis pulp-capping material thus prepared, the external stimuli to the nerves (pulp) can be prevented because it can directly cover a pulp-exposed portion, and the differentiation-stimulating effect into odontoblasts and the dentinogenesis-inducing effect can be effectively achieved because it can retain and maintain the HMG-CoA reductase inhibitor, an active ingredient, around the pulp-exposed portion for a long period of time.

### (III) Method of Producing Odontoblasts and the Application thereof

The present invention also provides a method of producing odontoblasts. The method is carried out by culturing cells (odontoblast precursors) capable of differentiating into odontoblasts, specifically, dental pulp stem cells, stem cells from apical papilla, or odontoblast precursor cells, in the presence of the HMG-CoA reductase inhibitor.

The term "odontoblast" used herein means a matured cell that secretes matrix components of dentin. In contrast, a slightly undifferentiated cell that is not a fully matured odontoblast but has the potential to differentiate into an odontoblast is referred to as an "odontoblast precursor cell". Additionally, the term "dental pulp stem cell" indicates a multipotent undifferentiated mesenchymal stem cell found in pulp tissues. Usually, the dental pulp stem cell can be isolated by culturing pulp tissues in a stem cell specific medium. In other words, these cells are classified as follows based on the degree of differentiation: ((1) minimum degree of differentiation (undifferentiated): dental pulp stem cell, stem cells from apical papilla, (2) moderate degree of differentiation: odontoblast precursor cell, (3) maximum degree of differentiation (maturity): odontoblast).

In the present invention, single cells consisting of one type of cells or a cell mixture consisting of two or more types of cells may be cultured as cells capable of differentiating into odontoblasts (in the present invention, cells including the above-mentioned dental pulp stem cells, stem cells from apical papilla, and odontoblast precursor cells are collectively termed "odontoblast precursors"). Origins of "odontoblast precursors" are not particularly limited; however, mammals are preferred. Specific examples may include rats, mice, rabbits, monkeys, pigs, dogs, and humans, with monkeys and humans being preferable and humans being more preferable.

"Odontoblast precursors" can be obtained from the extracted teeth of these mammals. For example, human pulp cells can be obtained in accordance with the method of About I. et al. (About I. et al.: Human dentin production in vitro. Exp Cell Res., 258:33-41, 2000). Additionally, human "odontoblast precursor cells" can be obtained from pulp tissues, for example, in accordance with the method of Gronthos S. et al. (Gronthos S. et al.: Postnatal human dental pulp stem cells (DPSCs) in vitro and in vivo. Proc. Natl. Acad. Sci. USA, 97:13625-30, 2000).

Specifically, human "odontoblast precursor" can be obtained in accordance with the following method:
(1) first, extracting an impacted tooth in an aseptic manner, and store the tooth in a suitable preservation solution, such as phosphate buffered saline (hereinafter abbreviated as PBS) solution;

(2) removing a calcified portion of the teeth, making the tissue into small pieces with a scalpel, and washing the tissue by using PBS solution, etc;

(3) next, treating the tissue with enzymes such as collagenase and dispase;

(4) after enzyme treatment, collecting cells by pipetting and centrifugation;

(5) examining the multipotential and proliferative potential of the collected cells, and confirming that "odontoblast precursors" are contained in these collected cells.

Cultivation of "odontoblast precursors" may be performed using a usual serum-containing culture medium or serum-free culture medium used to culture animal cells, under usual animal cell culture conditions (for example, in an incubator of a temperature ranging from a room temperature to 37°C and containing 5% CO₂, etc.). It is also possible to add additives such as ascorbic acid to the culture medium. The form of cultivation is not particularly limited; for example, cells may be cultured by static culture.

The concentration of HMG-CoA reductase inhibitor used for culturing the "odontoblast precursors" is 0.1 to 10 µM in the culture medium, preferably 0.2 to 5 µM, more preferably 0.5 to 2 µM.

Any HMG-CoA reductase inhibitor may be used insofar as it has an effect of inhibiting the effects of HMG-CoA reductase and thereby preventing the production of mevalonic acid in the cholesterol synthesis pathway in the body. As described above, specific examples may include pravastatin (brand name: Mevalotin), simvastatin (brand name: Lipovas), fluvastatin (brand name: Lescol), atorvastatin (brand name: Lipitor), rosuvastatin (brand name: Crestor), pitavastatin (brand name: Livalo), mevastatin, lovastatin, and salts thereof.

"Odontoblast precursors" may be cultured on a carrier. A preferable carrier is one that is durable as a scaffold for culturing the precursors for a period of time required for the formation of odontoblasts and subsequent dentinogenesis. A further preferable carrier is one that is biodegradable. Additionally, a carrier preferably consists of ingredients having compatibility with "odontoblast precursors".

No limit is given to the types of elements of the carrier. However, examples may include synthetic polymer materials such as ceramic base materials (for example, hydroxyapatite (HAp), tricalcium phosphate (β-TCP), tetracalcium phosphate, polyglycolic acid (PGA), poly(DL-lactide-co-glycolide) (PLGA), polylactic acid (PLA), polycaprolactone, etc., or protein materials such as collagen, gelatin, fibrin, etc.; or naturally derived materials such as hyaluronic acid and its salts, alginic acid and its salts, ivory, coral, etc. Note that when synthetic materials such as PGA, PLLA, PLGA, or polycaprolactone are used, these materials may be used by coating the surface with a collagen solution or fibronectin solution in order to improve the adhesion and proliferation of the cells.

The forms of the carrier may include a meshed form, sponge form, gel form, nonwoven form, particle form, etc. A preferable carrier is one that is formed in a shape in which odontoblast precursors are easily transplanted. A carrier having a platelike or spherical porous body or hollow body with one end open to allow blood vessels to easily penetrate thereinto from the surrounding is also preferable.

When culturing using a carrier, the method of the present invention may include the following three steps:
(i) step of culturing isolated "odontoblast precursors" (primary cultivation);
(ii) step of culturing cells obtained by primary cultivation described above by seeding the cells on a carrier (secondary cultivation); and
(iii) step of culturing the cells on the above-described carrier in the presence of HMG-CoA reductase inhibitor (differentiation induction).

In the above-described (i) primary cultivation step, cultivation is performed in order to obtain the necessary number of cells for regeneration of dentin in the subsequent steps. Cultivation of the "odontoblast precursors" in step (i) may be performed using a usual serum-containing culture medium or serum-free culture medium used to culture animal cells, under usual animal cell culture conditions (for example, cultivation in an incubator of a temperature ranging from a room temperature to 37°C and containing 5% CO₂, etc.). The form of cultivation is not particularly limited; for example, cells may be cultured by static culture. Additionally, the cells obtained by step (i) can be frozen-stored, and the cells that are thawed and proliferated as needed may be used for the operation of the above-described step (ii) and thereafter.

The above-described step (ii) is a step of proliferating "odontoblast precursors" on the carrier. Cells that were seeded in the carrier are attached to the carrier and proliferated. Cultivation of the "odontoblast precursors" in step (ii) may also be performed using a usual serum-containing culture medium or serum-free culture medium used to culture animal cells, under usual animal cell culture conditions (for example, cultivation in an incubator of a temperature ranging from a room temperature to 37°C and containing 5% CO₂, etc.). The cultivation period is preferably 1 to 30 days, more preferably 5 to 25 days.

The above-described step (iii) is a step of inducing differentiation into odontoblasts. The amount of HMG-CoA reductase inhibitor used is as described above: 0.1 to 10 µM, preferably 0.2 to 5 µM, more preferably 0.5 to 2 µM, in terms of concentration in the culture medium.

Dentin can be formed using odontoblasts thus prepared. For example, it is known that dental pulp stem cells produce dentin when transplanted (Gronthos S., Mankani M., Brahim J., Robey PG, Shi S. Postnatal human dental pulp stem cells (DPSCs) in vitro and in vivo. Proc. Natl. Acad. Sci. USA., 97:13625-13630, 2000).

Thus, the transplantation of the odontoblasts, which are differentiated by the method of the present invention as dental treatment material, into a patient with dental disease such as caries can help treat such a patient with dental disease. In this case, odontoblasts obtained by the method of the present invention may be transplanted with a carrier directly into a patient. In this way, dentin can be regenerated at a transplant site (opening of pulp chamber and pulp-exposed portion) of the patient.

It may also be possible, according to needs, to temporarily transplant odontoblasts differentiated by the method of the present invention with a carrier into a mammal other than humans so as to regenerate dentin in the body of the recipient animal, and then to transplant the regenerated dentin, as dental treatment material, into a site where tooth structure is lost such as a caries portion. Although there are no particular limits on the kind of recipient animals, immunodeficient rodents such as mice (for example, nude mice), rats (for example, nude rats), etc. may be preferably used. The odontoblasts can also be transplanted as an artificial root composed of dentin into the alveolar bone of a human patient having a defective tooth due to a dental disease. In this case, an artificial tooth root may be made in advance by forming dentin on the surface of biomaterials in tooth-root form such as hydroxyapatite, titanium, etc., using cell culturing or transplantation. Note that, there are no particular limits as to the transplant site of a mammal other than humans: examples include a site under the skin of the back.

### [Examples]

Below, the present invention is described in more detail with reference to Examples, but the present invention is not limited to these Examples.

### Example 1

### (1) Isolation and Cultivation of Dental Pulp Stem Cells (DPSCs)

Dental pulp stem cells (DPSCs) were isolated in accordance with a method of Gronthos et al. (Proc. Natl. Acad. Sci. USA, 97: 13625-30, 2000) from a human extracted tooth that was submitted with informed consent under the permission of Okayama University Dental School Ethics Committee.

Specifically, pulp obtained from an extracted human tooth was enzyme-treated for 30 minutes at 37°C with 3 mg/ml type 1 collagenase (Worthington Biochemicals Corp.) and 4 mg/ml dispase (Roche Diagnostic/Boehringer Mannheim Corp.), and then cultured using α-MEM culture medium containing 10% fetal bovine serum (FBS), L-ascorbic acid (0.1 mM), L-glutamine (2 mM), penicillin (100 µg/ml), and streptomycin (100 µg/ml).

For the DPSCs subcultured for 2 to 10 generations, the RT-PCR method was used to observe expression of the dentin sialophosphoprotein (DSPP) gene, i.e., the odontoblast-specific marker (Narayanan K. et al., J. Biol. Chem., 281(28):19064-71, 2006).

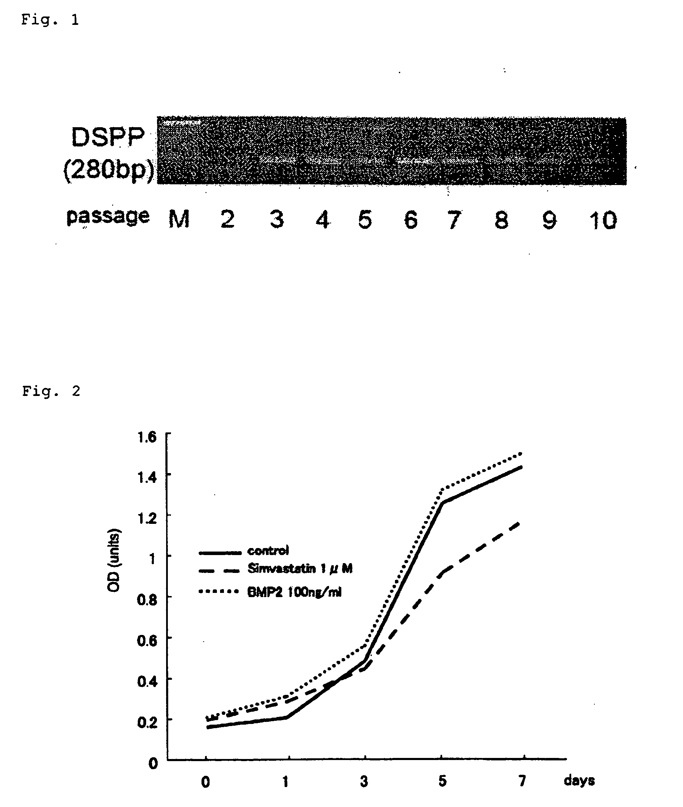

Fig. 1 shows the results of RT-PCR. Based on the amount of expression of DSPP in dentin dental pulp stem cells, cells subcultured for 3 to 5 generations were used for the following experiments.

### (2) Effect of HMG-CoA Reductase Inhibitor on Cell Proliferation

Simvastatin (SVS) (Calbiochem Inc.) was used as a HMG-CoA reductase inhibitor to investigate the effect of HMG-CoA reductase inhibitor on the proliferation of dental pulp stem cells (DPSCs).

Specifically, the DPSCs prepared above (1) were cultured under 5% CO₂ at 37°C in α-MEM culture medium including 10% fetal bovine serum (FBS) containing SVS at a concentration of 1 µM, L-ascorbic acid (0.1 mM), L-glutamine (2 mM), penicillin (100 µg/ml), and streptomycin (100 µg/ml). The culture medium was exchanged twice a week, and the number of cells on day 1, day 3, day 5 and day 7 was indirectly evaluated using the MTS method (Barltrop, J.A. et al. 5-(3-carboxymethoxyphenyl)-2-(4,5-dimenthylthiazoly)-3-(4-sulfophenyl)tetrazolium, inner salt (MTS) and related analogs of 3-(4,5-dimethylthiazolyl)-2,5-diphenyl- tetrazolium bromide (MTT) reducing to purple water-soluble formazans as cell-viability indicators. Bioorg. & Med. Chem. Lett. 1, 611, 1991).

Note that DPSCs were cultured in a similar manner as described above and the number of cells was evaluated using α-MEM culture medium as a control including 10% fetal bovine serum (FBS) containing PBS instead of SVS, L-ascorbic acid (0.1 mM), L-glutamine (2 mM), penicillin (100 µg/ml), and streptomycin (100 µg/ml); and another α-MEM culture medium as a comparison including 10% fetal bovine serum (FBS) containing BMP-2 (bone morphogenetic protein) (R&D systems) known to have a dentinogenesis inducing effect at a concentration of 100 ng/ml, L-ascorbic acid (0.1 mM), L-glutamine (2 mM), penicillin (100 µg/ml), and streptomycin (100 µg/ml).

Fig. 2 shows the results. As can be seen from Fig. 2, on day 5 from the beginning of cultivation, cultivation in the presence of BMP-2 at a concentration of 100 ng/ml showed a significant increase in the proliferation of dental pulp stem cells (DPSCs) (1.05-fold, p<0.0070), whereas cultivation in the presence of SVS at a concentration of 1 µM showed a significant decrease in the proliferation of dental pulp stem cells (DPSCs) (0.73-fold, p<0.0001). This revealed that simvastatin, which is an HMG-CoA reductase inhibitor, has an effect of inhibiting proliferation of dental pulp stem cells (DPSCs).

### (3) Effect of HMG-CoA Reductase Inhibitor on Cellular Differentiation

Simvastatin (SVS) (Calbiochem Inc.) was used as an HMG-CoA reductase inhibitor to investigate the effect of the HMG-CoA reductase inhibitor on the cellular differentiation of dental pulp stem cells (DPSCs) into odontoblasts.

Specifically, the DPSCs prepared above (1) were cultured under a 5% CO₂ gas phase at 37°C in α-MEM culture medium including 10% fetal bovine serum (FBS) containing SVS at a concentration of 1 µM, L-ascorbic acid (0.1 mM), L-glutamine (2 mM), penicillin (100 µg/ml), and streptomycin (100 µg/ml). Total RNA was collected from cells obtained on day 3, day 7, and day 10 from the beginning of cultivation, and gene expression of dentin sialophosphoprotein (DSPP), i.e., an odontoblast-specific marker (Ritchie HH et al. J. Biol. Chem., 269:3698-702, 1994; Butler WT et al. Matrix., 12:343-51, 1992), and gene expression of osteocalcin (OCN), i.e., a marker of calcification (Bronckers AL et al. Connect Tissue Res., 22:65-70, 1989) were evaluated using qualitative RT-PCR (Narayanan K et al., J Biol. Chem., 281(28):19064-71, 2006).

Note that 10% Dulbecco's modified Eagle medium containing PBS instead of SVS was used as a control and 10% Dulbecco's modified Eagle medium containing BMP-2 (R&D systems) at a concentration of 100 ng/ml instead of SVS was used as a comparison so as to culture DPSCs in a similar manner as described above, and gene expressions of DSPP and OCN were evaluated.

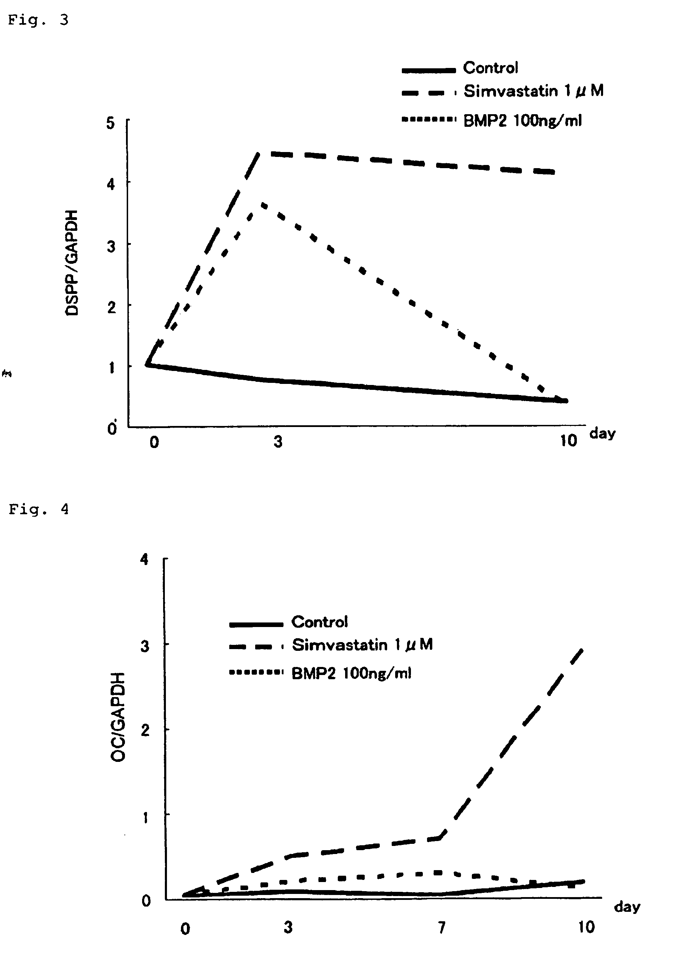

Fig. 3 shows the results indicating gene expression of DSPP, and Fig. 4 shows the results indicating gene expression of OCN.

As is clear from these figures, cultivation of dental pulp stem cells (DPSCs) in SVS-containing culture medium resulted in a significant increase in gene expression of DSPP that is an odontoblast-specific marker (10.9-fold, p=0.0111). Additionally, gene expression of OCN that is a marker of calcification was also significantly increased compared with the control (18.8-fold, p=0.0004) and the BMP-2 (7.4-fold, p=0.0002) (Scheffe's multiple comparison test).

Based on the above, it became clear that simvastatin, which is an HMG-CoA reductase inhibitor, has an effect of inhibiting cell proliferation of dental pulp stem cells (DPSCs) and inducing differentiation of DPSCs into odontoblasts.

### Example 2

As an HMG-CoA reductase inhibitor, in addition to simvastatin (Calbiochem) used in Example 1, mevastatin (Toronto Research Chemicals Inc.), fluvastatin (Calbiochem), lovastatin (SIGMA), pitavastatin (Toronto Research Chemicals Inc.), and plavastatin (Daiichi Sankyo Company, Limited) were used to conduct experiments similar to Example 1, sections (2) and (3).

### (1) Effect of HMG-CoA Reductase Inhibitor on Dental Pulp Stem Cells (DPSCs)

DPSCs were cultured in a manner similar to Example 1, section (2), using simvastatin (1 µM), mevastatin (1 µM), fluvastatin (1 µM), lovastatin (1 µM), pitavastatin (1 µM), and plavastatin (200 µM) respectively as HMG-CoA reductase inhibitors. On day 0, day 3, and day 5 from the beginning of cultivation, the cell number of DPSCs was measured using the MTS method. A control was also prepared in a manner similar to Example 1, section (2), and an experiment was carried out.

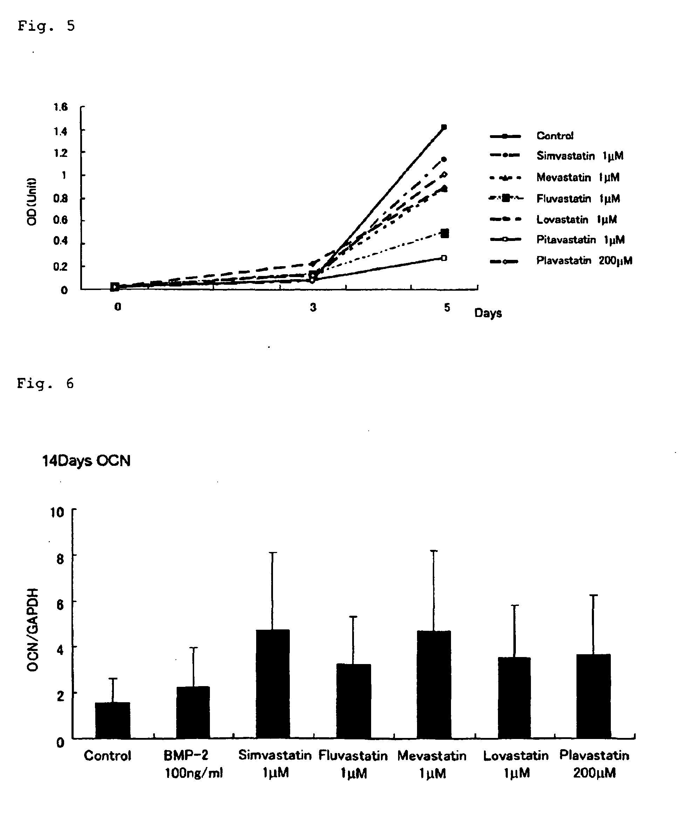

Fig. 5 shows the results. As is clear from Fig. 5, on day 5 from the beginning of cell cultivation, fat-soluble statins (simvastatin, mevastatin, fluvastatin, lovastatin, pitavastatin) inhibited proliferation of DPSCs with a significant difference at a concentration of 1 µM, compared with the control. Additionally, observation showed that a water-soluble statin (pravastatin) inhibited proliferation of DPSCs at a concentration of 200 µM.

### (2) Effect of HMG-CoA Reductase Inhibitor on Cellular Differentiation

Dental pulp stem cells (DPSCs) were cultured in a manner similar to Example 1, section (3), using simvastatin (1 µM), fluvastatin (1 µM), mevastatin (1 µM), lovastatin (1 µM), and plavastatin (200 µM) respectively as HMG-CoA reductase inhibitors. Gene expression of OCN, i.e., a marker of calcification was evaluated on day 14, using qualitative RT-PCR. Additionally, a control and a comparison (BMP-2) were also prepared in a manner similar to Example 1, section (3).

Fig. 6 shows the results. As is clear from Fig. 6, on day 14 from the beginning of cell cultivation, it was observed that treating cells using fat-soluble statins (simvastatin, mevastatin, fluvastatin, lovastatin) and a water-soluble statin (pravastatin) resulted in an increase in gene expression of OCN, which is a marker of calcification, compared with the control and the comparison (BMP-2).

The above indicated that not only the simvastatin observed in Example 1 but also all other substances containing an HMG-CoA reductase inhibitor have an effect of inhibiting cell proliferation of dental pulp stem cells (DPSCs) and inducing differentiation of DPSCs into odontoblasts.

### Example 3

(1) Fasudil (Asahi Kasei Corporation), which is a substance having an effect of inhibiting Rho/Rho-kinase just like an HMG-CoA reductase inhibitor, was used to examine an effect thereof on proliferation of dental pulp stem cells (DPSCs).

Specifically, simvastatin (Calbiochem) (1 µM) as an HMG-CoA reductase inhibitor and the above-mentioned Fasudil (1 µM, 10 µM, and 100 µM) respectively were used to culture DPSCs in a manner similar to Example 1, section (2). On day 5 from the beginning of cultivation, the cell number of DPSCs was measured using the MTS method. A control was also prepared in a manner similar to Example 1, section (2), and an experiment was carried out.

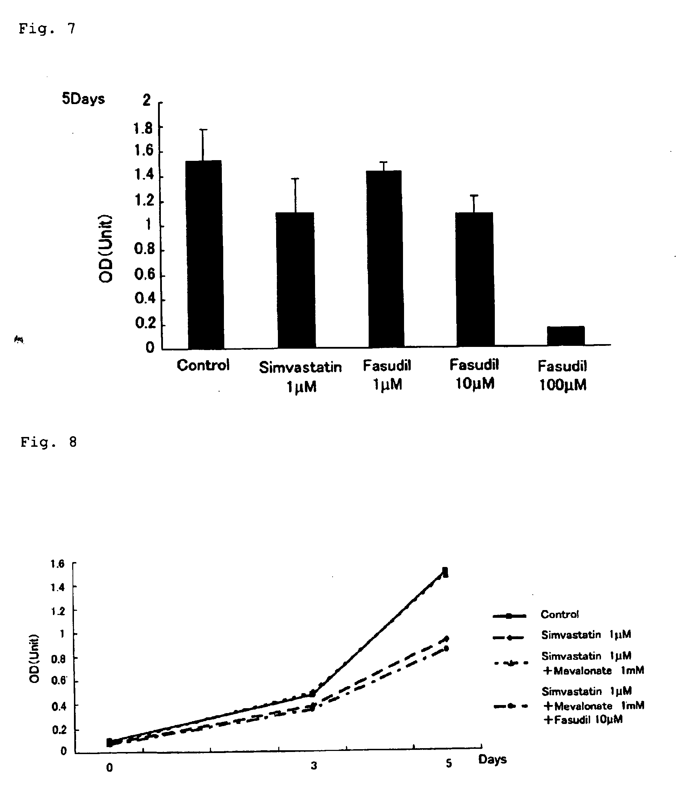

Fig. 7 shows the results. As is clear from Fig. 7, on day 5 from the beginning of cell cultivation, Fasudil at a concentration of not less than 10 µM inhibited proliferation of DPSCs.

(2) Simvastatin (Calbiochem) was used as an HMG-CoA reductase inhibitor to examine how inhibition of the proliferation of dental pulp stem cells (DPSCs) induced by simvastatin (see Example 1, section (2)) would be affected by the supply of mevalonate or Fasudil (Asahi Kasei Corporation).

Note that mevalonate is a molecule produced in vivo from HMG-CoA through the effects of an HMG-CoA reductase in the cholesterol synthesis pathway in the body. In other words, mevalonate is a molecule in vivo whose production from HMG-CoA is inhibited by an HMG-CoA reductase inhibitor. Additionally, just like a HMG-CoA reductase inhibitor, Fasudil is a substance having an effect of inhibiting Rho/Rho-kinase.

Specifically, in Example 1, section (2), dental pulp stem cells (DPSCs) were cultured in the presence of each of the following: (a) simvastatin (1 µM) (simvastatin added group); (b) simvastatin (1 µM) and mevalonate (1 mM) (mevalonate added group); and (c) simvastatin (1 µM), mevalonate (1 mM) and Fasudil (10 µM) (Fasudil combined use group). On day 0, day 3, and day 5 from the beginning of cultivation, the cell number of DPSCs was measured using the MTS method. Additionally, a control was prepared by using PBS instead of the above-described test substances in a manner similar to Example 1, section (2), and tested in a similar manner as described above.

Fig. 8 shows the results. As is clear from Fig. 8, on day 5 from the beginning of cultivation, the (b) mevalonate added group showed the disappearance of the cell proliferation inhibiting effect due to the addition of HMG-CoA reductase inhibitor. Additionally, as is the case with the (a) simvastatin added group, the (c) Fasudil combined use group in which Fasudil (10 µM) was added in addition to mevalonate (1 mM) resulted in a greater inhibition of cell proliferation of DPSCs than the comparison, even though mevalonate was contained.

The experimental results of (1) and (2) show that inhibition of the production of mevalonate by an HMG-CoA reductase inhibitor leads to the inhibition of cell proliferation of DPSCs.

In other words, the results of Examples 1 to 3 indicate that a substance having an effect of inhibiting the effects of HMG-CoA reductase and thereby preventing the production of mevalonate in the cholesterol synthesis pathway in the body, i.e., an HMG-CoA reductase inhibitor, is useful as an agent for stimulating odontoblast differentiation and as a dentin regeneration inducer.

### Example 4 Examination of the Effect of BMP on the Inhibition of Cellular Proliferation

In accordance with the method of Example 1, section (2), dental pulp stem cells (DPSCs) were cultured in the presence of each of the following: (a) HMG-CoA reductase inhibitor (statin added group), (b) BMP-2, which is known to be induced by an HMG-CoA reductase inhibitor (BMP-2 added group), (c) Noggin (R&D systems), i.e., a BMP inhibitor (Noggin added group), (d) HMG-CoA reductase inhibitor and Noggin (statin-Noggin added group), and (e) BMP-2 and Noggin (BMP-2-Noggin added group); and the cell number of DPSCs was measured on day 0, day 3, and day 5 from the beginning of cultivation using the MTS method.

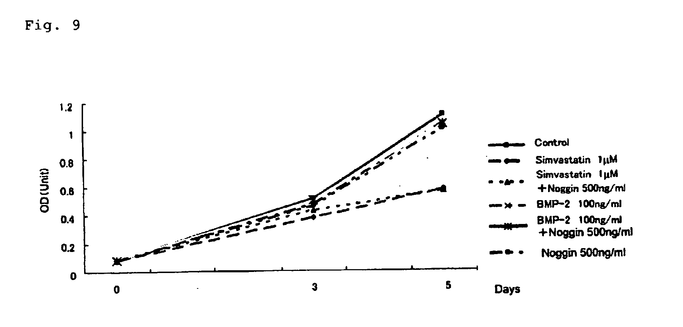

Fig. 9 shows the results. On day 5 from the beginning of cultivation, an effect on cellular proliferation of DPSCs was not observed in the BMP added group and the Noggin added group, compared with the control. Further, inhibition of cellular proliferation of DPSCs was observed in the statin added group and the statin-Noggin added group. Based on these observations, it appears that inhibition of cellular proliferation of DPSCs occurs through a pathway independent of statin-induced BMP-2.

As an example of the clinical application of the present invention, it is possible to impregnate a carrier such as a collagen gel or filling materials used in the dental field with an HMG-CoA reductase inhibitor so as to apply such impregnated materials to the exposed pulp surface or fill the exposed pulp surface with such impregnated materials. By so doing, dental pulp cells can be induced to differentiate into odontoblast and, as a result, regeneration of dentin can be promoted.

### [Brief Description of Drawings]

Fig. 1 shows the results of RT-PCR measurement of the expression of a odontoblast-specific marker (DSPP) by subculturing dental pulp stem cells (DPSCs).
Fig. 2 shows the results obtained by culturing dental pulp stem cells in the presence of an HMG-CoA reductase inhibitor (expressed as "statin" in this and the following drawings); in the presence of another dentinogenesis inducer (BMP-2); and under control conditions (control), and comparing the effect on cell proliferation under each condition.
Fig. 3 shows the results obtained by culturing dental pulp stem cells in the presence of an HMG-CoA reductase inhibitor (statin); in the presence of another dentinogenesis inducer (BMP-2); and under control conditions (control), separately, and measuring the effect on gene expression of DSPP (dentin sialophosphoprotein) under each condition.
Fig. 4 shows the results obtained by culturing dental pulp stem cells in the presence of an HMG-CoA reductase inhibitor (statin); in the presence of another dentinogenesis inducer (BMP-2); and under control conditions (control), separately, and measuring the effect on the gene expression of OCN (osteocalcin) under each condition.
Fig. 5 shows the results obtained by culturing dental pulp stem cells in the presence of various HMG-CoA reductase inhibitors (simvastatin (1 µM; Calbiochem Inc.), mevastatin (1 HGMM; Calbiochem Inc.), fluvastatin (1 µM; Calbiochem Inc.), lovastatin (1 µM; Sigma Corporation), pitavastatin (1 µM; Toronto Research Chemicals Ins.), and plavastatin (200 µM; Daiichi Sankyo Company Limited.)); and under control conditions (control), and comparing the effect on cell proliferation under each condition (Example 2).
Fig. 6 shows the results obtained by culturing dental pulp stem cells in the following conditions: in the presence of various HMG-CoA reductase inhibitors (simvastatin (1 µM; Calbiochem Inc.), mevastatin (1 µM; Calbiochem Inc.), fluvastatin (1 µM; Calbiochem Inc.), lovastatin (1 HGMM; Sigma Corporation), and plavastatin (200 µM; Daiichi Sankyo Company Limited.)) in the presence of another dentinogenesis inducer (BMP-2); and under control conditions (control), separately, and comparing the effect on the gene expression of OCN (osteocalcin) under each condition.
Fig. 7 shows the results obtained by culturing dental pulp stem cells in the presence of an HMG-CoA reductase inhibitor (simvastatin (1 µM; Calbiochem Inc.)); and in the presence of Fasudil, i.e., a Rho/Rho kinase inhibitor (1 µM, 10 µM, 100 µM), and comparing the effect on cell proliferation under each condition (Example 3, section (1)).
Fig. 8 shows the results obtained by culturing dental pulp stem cells in the presence of an HMG-CoA reductase inhibitor (simvastatin (1 µM; Calbiochem Inc.)); in the presence of the HMG-CoA reductase inhibitor and mevalonate; and in the presence of the HMG-CoA reductase inhibitor, mevalonate and Fasudil, i.e., a Rho-kinase inhibitor (1 µM, 10 µM, 100 µM), and comparing the effect on cell proliferation under each condition (Example 3, section (2)).
Fig. 9 shows the results obtained by culturing dental pulp stem cells in the presence of an HMG-CoA reductase inhibitor (Simvastatin (1 µM; Calbiochem Inc.)); in the presence of the HMG-CoA reductase inhibitor and Noggin; in the presence of BMP-2; in the presence of BMP-2 and Noggin; or in the presence of Noggin, and comparing the effect on cell proliferation under each condition (Example 4).

## Claims

1. A HMG-CoA reductase inhibitor for use as a dentinogenesis inducer.

2. The HMG-CoA reductase inhibitor for use as a destriogenesis induces according to claim 1, wherein said HMG-CoA reductase inhibitor is for use as a dentinogenesis pulp-capping material.

3. The HMG-CoA reductase inhibitor inhibitor for use as a destriogenesis induces according to claim 1, wherein said HMG-CoA reductase inhibitor is for use in caries treatment.

4. The HMG-CoA reductase inhibitor inhibitor for use as a destriogenesis induces according to claim 3, wherein said HMG-CoA reductase inhibitor is used for filling restoration of a carious portion and/or an exposed pulp surface or said HMG-CoA reductase inhibitor is used as tooth coating material for an exposed pulp surface.

5. The HMG-CoA reductase inhibitor inhibitor for use as a destriogenesis induces according to any one of claims 1 to 4, wherein said HMG-CoA reductase inhibitor is at least one selected from the group consisting of pravastatin, simvastatin, fluvastatin, atorvastatin, rosuvastatin, mevastatin, lovastatin, pitavastatin, and salts thereof.

6. A method of producing an odontoblast comprising a step of culturing a cell capable of differentiating into an odontoblast in the presence of an HMG-CoA reductase inhibitor.

7. The method of producing an odontoblast according to claim 6, wherein the cell capable of differentiating into an odontoblast is at least one member selected from the group consisting of dental pulp stem cells, stem cells from apical papilla, and odontoblast precursor cells.

8. The method of producing an odontoblast according to claim 6, wherein the culturing is performed in the presence of an HMG-CoA reductase inhibitor at a concentration of 0.1-10 pM.

9. The method of producing an odontoblast according to claim 6, wherein the HMG-CoA reductase inhibitor is at least one selected from the group consisting of pravastatin, simvastatin, fluvastatin, atorvastatin, rosuvastatin, mevastatin, lovastatin, pitavastatin, and salts thereof.

## Patentansprüche

1. Ein HMG-CoA Reduktase Inhibitor zur Verwendung als Dentinogenese Induktor.

2. Der HMG-CoA Reduktase Inhibitor zur Verwendung als Dentinogenese Induktor nach Anspruch 1, wobei besagter HMG-CoA Reduktase Inhibitor für die Verwendung als Material zur Pulpaüberkappung für die Dentinogenese dient.

3. Der HMG-CoA Reduktase Inhibitor zur Verwendung als Dentinogenese Induktor nach Anspruch 1, wobei besagter HMG-CoA Reduktase Inhibitor für die Verwendung in der Kariesbehandlung dient.

4. Der HMG-CoA Reduktase Inhibitor zur Verwendung als Dentinogenese Induktor nach Anspruch 3, wobei besagter HMG-CoA Reduktase Inhibitor zur Füllung zur Wiederherstellung eines kariösen Abschnittes und/oder einer freiliegenden Pulpaoberfläche verwendet wird, oder besagter HMG-CoA Reduktase Inhibitor als Zahnbeschichtungsmaterial für eine freiliegende Pulpaoberfläche verwendet wird.

5. Der HMG-CoA Reduktase Inhibitor zur Verwendung als Dentinogenese Induktor nach einem der Ansprüche 1 bis 4, wobei besagter HMG-CoA Reduktase Inhibitor mindestens einer ausgewählt aus der Gruppe bestehend aus Pravastatin, Simvastatin, Fluvastatin, Atorvastatin, Rosuvastatin, Mevastatin, Lovastatin, Pitavastatin , und Salzen davon ist.

6. Ein Verfahren zur Herstellung eines Odontoblasten, umfassend einen Schritt der Kultivierung einer Zelle, die in Gegenwart eines HMG-CoA Reduktase Inhibitors zur Differenzierung in einen Ontoblasten fähig ist.

7. Das Verfahren zur Herstellung eines Odontoblasten nach Anspruch 6, wobei die Zelle, die zur Differenzierung in einen Odontoblasten fähig ist, mindestens ein Mitglied ausgewählt aus der Gruppe bestehend aus Zahnpulpastammzellen, Stammzellen der apicalen Papilla, und Ondoblasten-Vorläuferzellen ist.

8. Das Verfahren zur Herstellung eines Odontoblasten nach Anspruch 6, wobei die Kultivierung in Gegenwart eines HMG-CoA Reduktase Inhibitors in einer Konzentration von 0.1-10 pM durchgeführt wird.

9. Das Verfahren zur Herstellung eines Odontoblasten nach Anspruch 6, wobei der HMG-CoA Reduktase Inhibitor mindestens einer ausgewählt aus der Gruppe bestehend aus Pravastatin, Simvastatin, Fluvastatin, Atorvastatin, Rosuvastatin, Mevastatin, Lovastatin, Pitavastatin , und Salzen davon ist.

## Revendications

1. Inhibiteur de l'HMG-CoA réductase pour son utilisation comme agent d'induction de la dentinogenèse.

2. Inhibiteur de l'HMG-CoA réductase pour son utilisation comme agent d'induction de la dentinogenèse selon la revendication 1, dans lequel ledit inhibiteur de l'HMG-CoA réductase est destiné à être utilisé en tant que matériau de coiffage pulpaire pour la dentinogenèse.

3. Inhibiteur de l'HMG-CoA réductase pour son utilisation comme agent d'induction de la dentinogenèse selon la revendication 1, dans lequel ledit inhibiteur de l'HMG-CoA réductase est destiné à être utilisé dans le traitement des caries.

4. Inhibiteur de l'HMG-CoA réductase pour son utilisation comme agent d'induction de la dentinogenèse selon la revendication 3, dans lequel ledit inhibiteur de HMG-CoA réductase est utilisée pour le remplissage de restauration d'une partie cariée et/ou d'une surface pulpaire exposée ou ledit inhibiteur de HMG-CoA réductase est utilisée comme matériau de revêtement dentaire pour une surface pulpaire exposée.

5. Inhibiteur de l'HMG-CoA réductase pour son utilisation comme agent d'induction de la dentinogenèse selon l'une quelconque des revendications 1 à 4, dans lequel ledit inhibiteur de l'HMG-CoA réductase est au moins un élément sélectionné dans le groupe consistant en la pravastatine, la simvastatine, la fluvastatine, l'atorvastatine, la rosuvastatine, la mévastatine, la lovastatine, la pitavastatine et leurs sels.

6. Procédé de production d'un odontoblaste comprenant une étape de culture d'une cellule capable de se différencier en un odontoblaste en présence d'un inhibiteur de l'HMG-CoA réductase.

7. Procédé de production d'un odontoblaste selon la revendication 6, dans lequel la cellule capable de se différencier en un odontoblaste est au moins un élément choisi dans le groupe constitué de cellules souches de la pulpe dentaire, de cellules souches des papilles apicales, et de cellules précurseurs odontoblastiques.

8. Procédé de production d'un odontoblaste selon la revendication 6, dans lequel la culture est effectuée en présence d'un inhibiteur de l'HMG-CoA réductase à une concentration de 0,1-10 pM.

9. Procédé de production d'un odontoblaste selon la revendication 6, dans lequel l'inhibiteur de l'HMG-CoA réductase est au moins un élément sélectionné dans le groupe constitué de la pravastatine, de la simvastatine, de la fluvastatine, de l'atorvastatine, de la rosuvastatine, de la mévastatine, de la lovastatine, de la pitavastatine et de leurs sels.
